(19) Europäisches Patentamt
European Patent Office
Office européen des brevets

(11) **EP 2 005 885 A1**

(12) **EUROPEAN PATENT APPLICATION**

(43) Date of publication:
**24.12.2008 Bulletin 2008/52**

(51) Int Cl.:
**A61B 5/103** $^{(2006.01)}$

(21) Application number: **07252478.8**

(22) Date of filing: **19.06.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Astron Clinica Limited**
**The Mount Toft**
**Cambridge, CB23 2RL (GB)**

(72) Inventors:
• **Cotton, Symon**
**Great Gransden SG19 3QS (GB)**

• **Morse, Robert**
**Cambridge CB1 3PU (GB)**
• **Chellingworth, Mark**
**Vale of Glamorgan**
**Wales CF64 2NP (GB)**

(74) Representative: **Fox, Nicholas Russell Philip et al**
**Ipulse**
**26 Mallinson Road**
**London SW11 1BP (GB)**

(54) **Method and apparatus for measuring skin texture**

(57) A first image of an area of an individual's skin (2) illuminated by a light source (3) via a polarizing filter (4) is obtained by a digital camera (1) via a second polarizing filter (5) arranged to filter visible light having the same polarity as light passing through the first polarizing filter (4). This image is then processed by a computer (6) which utilises the detected levels of light and a model (15-20) of the interaction of light with chromophores present in the skin (2) to determine expected levels light returned by the illuminated area of skin (2) on the basis of determined concentrations of chromophores present in the skin. A second image of the individual's skin (2) illuminated by the light source (3) in the absence of the polarizing filter (4) is then obtained. A surface map indicative of skin texture is then determined based of the differences between the first and second images scaled by geometry data indicative of variations in the strength of illumination due to variations in surface geometry of the individual's skin (2) derived from the difference between the first obtained image and the expected appearance of the illuminated are of skin (2) based on the calculated estimated concentrations of chromophores.

Fig.2.

**Description**

The present application relates to methods and apparatus for measuring skin texture. In particular embodiments of the present application concern methods and apparatus for measuring skin texture and identifying areas of dry skin.

When the skin is viewed in close up the surface is composed of fine lines and wrinkles. Detailed measurements of these structures are of great interest in both the research of products designed to reduce the appearance of wrinkles and also in the education of consumers. Standard techniques to measure the topology of skin range from making physical silicon replicas of the skin which are than traced to stereo and fringe projection. All of these techniques produce excellent results but are limited to laboratory analysis due to costs and acquisition times. An alternative system for measuring the detailed topology of the skin is therefore desirable.

In accordance with one aspect of the present invention there is provided a method of measuring skin surface texture comprising:

illuminating an area of skin with polarized light;
obtaining a first measurement of light returned by the illuminated area of skin wherein the measured light is light with a different polarity to the light with which said area of skin is illuminated;
processing the obtained measurement of light utilising a model of the interactions of light with chromophores present in the skin to determine variations in returned light levels arising due to variations in levels of illumination;
obtaining a second measurement of light returned by the illuminated area of skin wherein the measured light includes light of the same polarity as the light with which said area of skin is illuminated;
utilising the difference between said first and said second measurements of light returned by the illuminated area of skin and said determined variations in returned light levels arising due to variations in levels of illumination to obtain a measurement of the surface texture of the illuminated area of skin.

In accordance with another aspect of the present invention there is provided an apparatus for measuring skin surface texture, the apparatus comprising:

a light source operable to illuminate an area of skin with polarized light;
a detector operable to obtain a first and a second measurement of light returned by an illuminated area of skin wherein the first measurement of light is light of a different polarity to the light with which said light source is operable to illuminate said area of skin and said second measurement of light includes light of the same polarity as the light by said light source is operable to illuminate said area of skin; and
a processor operable to:

process an obtained first measurement of light utilising a model of the interactions of light with chromophores present in the skin to determine variations in returned light levels arising due to variations in levels of illumination; and
utilise the difference between detected first and said second measurements of light returned by the illuminated area of skin and determined variations in returned light levels arising due to variations in levels of illumination to obtain a measurement of the surface texture of the illuminated area of skin.

Further aspects and embodiments of the present invention will become apparent with reference to the accompanying drawings in which:

Figure 1 is a schematic cross sectional view through a layer of skin illustrating the structure of the skin and the interaction of that structure with incident light;
Figure 2 is a schematic block diagram of a skin texture measurement system in accordance with a second embodiment of the present invention;
Figure 3 is a flow diagram of the processing performed by the skin texture measurement system of Figure 2;
Figure 4A is an image of an eye of an individual; and
Figure 4B is an image of the eye of an individual of Figure 4A generated from utilising the estimated distributions and concentrations of blood an melanin represented by the original image in the absence of variations in appearance due to factors other than chromophore distributions.

Interaction of Light with the Skin

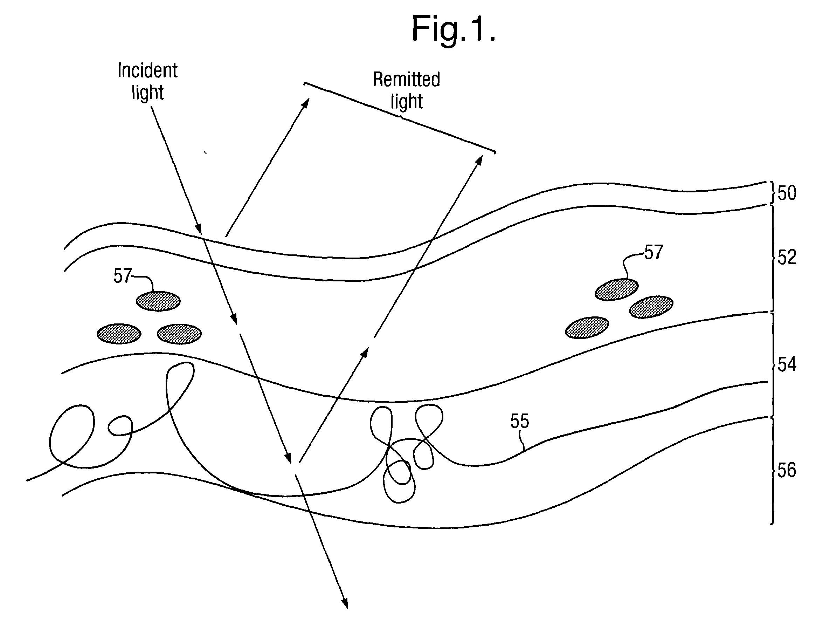

By way of background and to assist understanding, before describing embodiments of the present invention, the physical structure of skin and the interaction of skin with light will first be briefly explained with reference to Figure 1.

As shown in Figure 1, skin has a layered structure comprising an outer cornified layer 50 also known as the stratum corneum, the epidermis 52, and the dermis which itself can be divided into the papillary dermis 54 which contains the blood supply 55 for the skin and the reticular dermis 56.

When light is incident on the skin, much of the light is immediately reflected when coming into contact with the outer cornified layer 50. A proportion of incident light does, however, pass through the cornified layer 50 and proceeds to interact with the constituents of the epidermis 52 and the papillary dermis 54. As light passes through the epidermis 52 and the papillary dermis 54 the light is absorbed by various chromophores present in the skin, most notably chromophores such as haemoglobin present in the blood in blood vessels 55 in the papillary dermis, melanin, a pigment produced by melanocytes 57 in the epidermis 52 and collagen a fibrous material present throughout the skin. By the time the incident light reaches the reticular dermis 56 the scattering of light is highly forward and therefore for that reason the reticular dermis 56 can for all intents and purposes be considered returning no light.

In addition to chromophores present in the epidermis 52 and papillary dermis 54 absorbing various wavelengths, certain structures in the skin most notably collagen cause incident light to be reflected.
The interaction of light with collagen in the skin is such to cause the light to loose any original polarization. The outward appearance of the skin can therefore be considered to be a mixture of the light immediately reflected by the cornified layer 50 and the remitted light which has interacted with the chromophores present in the epidermis 52 and the papillary dermis 54.

As will be described, the present invention utilises the fact that the appearance of the skin is dependent upon the reflection of light from the surface of the skin and the interaction of light with structures and chromophores below the surface to obtain a measurement of the skin's surface texture.

Specific Embodiment

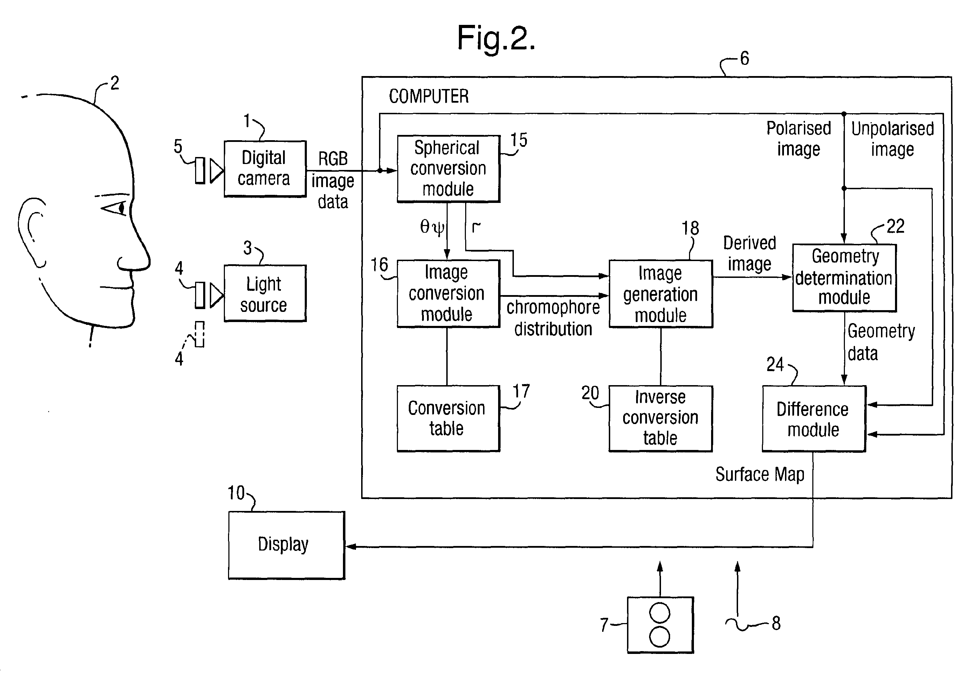

Referring to Figure 2 which is a schematic block diagram of an embodiment of the present invention, a skin texture measurement system is provided which comprises a conventional digital camera 1 which is arranged to obtain an image of an individual 2 illuminated by a light source 3.

A first 4 polarizer is then provided where the first polarizer is movable between a first position in front of the light source 3 which causes the light source 3 to illuminate an individual 2 with polarized light and a second position where the light source is able to illuminate the individual 2 without the light passing through the first polarizer 3. A second polarizer 5 is then provided in front of the lens of digital camera 5 with the two polarizers 4,5 being arranged so that the second polarizer 5 filters light polarized by the first polarizer 4.

The digital camera 1 is arranged to obtain images of an individual 2 illuminated by the light source 3 and then pass these images to a computer 6 which is configured by software either provided on a disk 7 or by receiving an electrical signal 8 by via a communications network to be configured into a number of functional modules. 15-24 which cause the computer 6 to process the image data received from the digital camera 1 to generate an output image which is shown on a display 10.

More specifically, two images of the surface of an individual are obtained. The first of these is obtained with the light source 3 illuminating the individual via the first polarizer 4, whereas the second image is obtained in the absence of the polarizer 4. These two images are then processed together with geometry data derived from the first image indicative of the extent the strength of illumination varies across the image to calculate a surface map image for display.

The functional modules illustrated in Figure 5 are purely notional in order to assist with the understanding of the working of the claimed invention and may not in certain embodiments directly correspond with blocks of code in the source code for the software. In other embodiments the function performed by the illustrated functional modules may be divided between different modules or may be performed by the re use of the same modules for different functions.

In the present embodiment the functional modules comprise a spherical conversion unit 15 for converting RGB image data into corresponding spherical co-ordinates, an image conversion module 16 and a conversion table 17 for processing spherical angular co-ordinates to generate data indicative of concentrations of blood and melanin; an image generation module 18 and an inverse conversion table 20 operable to generate image data utilising chromophores distribution data; a geometry determination module 22 for identifying variations in appearance in an image of an individual due to lighting variations and variations in surface geometry; and a difference module 24 for processing geometry data and images obtained from the digital camera 1 to generate a surface map for display on a display screen 10.

Processing of Obtained Image Data

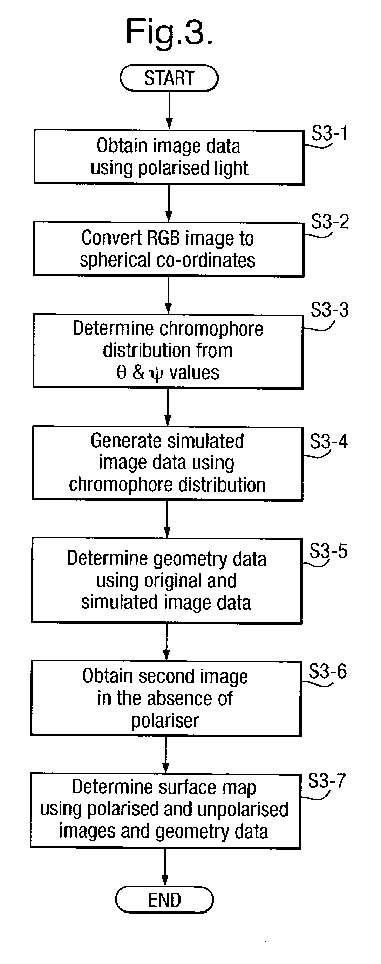

Referring to Figure 3 which is a flow diagram of the processing performed by the computer 6 of Figure 2, initially (S3-1) an image is obtained by the digital camera 1 of the individual 2 illuminated by the light source 3 with the first polarizer 4 positioned so that the individual 2 is illuminated by polarized light. The presence of the second polarizer 5 will then mean that the image obtained by the camera 1 will be dependent upon the interaction of the light with the underlying structures of the skin being imaged since any light reflected directly from the surface of the skin will be filtered by the second polarizer 5.

In this embodiment as the digital camera 1 comprises a conventional digital camera, the image data generated by the digital camera 1 comprises RGB values ranging from 0 to 255 for a large array of pixels where the RGB values are indicative of the extent light received by a photo receptor within the camera 1 for each pixel in an image appears to be red, green and blue where a completely black pixel has RGB values of 0, 0, 0 and a completely bright white pixel has RGB values of 255, 255, 255.

When an image of an individual 2 illuminated by polarized light has been obtained by the camera 12, the image is processed (s3-2-s3-5) to derive geometry data indicative of the variation in illumination arising due to large scale variations in surface geometry.

This processing is achieved by passing the obtained image to the spherical conversion module 15 which converts (S3-2) the conventional RGB data for each pixel in an image into a corresponding set of spherical co-ordinates θ ψ r where the spherical angles of θ ψ are substantially indicative of the hue and chromaticity represented by an individual pixel in an image captured by the digital camera 1 and the radial co-ordinate r is substantially indicative of the brightness of the pixel.

This conversion is achieved in a conventional manner with

$$\theta = \cos^{-1}\left( B\left( R^2 + B^2 + G^2 \right)^{-\frac{1}{2}} \right)$$

$$\psi = \tan^{-1}\left( \frac{G}{R} \right)$$

and

$$r = \left( R^2 + B^2 + G^2 \right)^{\frac{1}{2}}$$

The conversion is performed for each pixel in the original pixel array for the image generated by the digital camera. The result of the conversion is a set of spherical θ ψ r co-ordinates for each pixel in the original image.

The array of radial elements r is then passed directly to the image generation module 18 whereas arrays of the calculated angular spherical co-ordinates θ and ψ are in this embodiment passed to the image conversion module 16.

After the spherical conversion module 15 has converted the RGB values for an image into spherical co-ordinates, the image conversion module 16 then processes (s3-3) the generated array of θ and ψ values to obtain values indicative of the concentration of blood and melanin at individual points on the surface of the skin of the individual.

In this embodiment this is achieved by processing each pair of θ and ψ values for each pixel in an array in turn by scaling the θ and ψ values so that instead of comprising values between π and - π, and 0 and π/2, the scaled θ and ψ values comprise integer values ranging between 0 and 255. These scaled θ and ψ values are then utilised to access the conversion table 17 which in this embodiment is a 255 by 255 a lookup table associating pairs of scaled θ and ψ co-ordinates with pairs of concentrations of blood and melanin liable to give rise to such scaled θ and ψ values. In this embodiment, the conversion table 17 comprises a table associating blood and melanin concentrations with various θ and ψ values, where the θ and ψ values fall within the expected range of the colour space for skin. In the event that the combination of θ and ψ values for a particular pixel falls outside the range of values for which chromophores concentration data is stored within the conversion table 17, in this embodiment the conversion module 16 returns a null value for the concentration of blood and melanin for the pixel with θ and ψ values for the pixel.

After chromophore distribution values for blood and melanin for each of the pixels in an image have been calculated by the conversion module 16, this chromophore distribution data is then passed by the conversion module 12 to the image generation module 18. When the chromophore distribution values are received by the image generation module 18, the image generation module 18 then (s3-4) proceeds together with the inverse conversion table 20 to generate a derived image of the individual 2 indicative of the appearance of the individual to the extent that it is dependent upon the distribution of blood and melanin.

In this embodiment initially the image generation module 18 processes the received chromophore distribution data for each pixel in an image to generate a corresponding expected pair of θ and ψ colour angles. In this embodiment this conversion is achieved by the image generation module 18 accessing the inverse conversion table 20 which is a lookup table which associates each possible pair of determined blood and melanin concentrations for a pixel with a corresponding expected θ and ψ values. The inverse conversion table 20 is therefore data representative of an inverse function corresponding to the function for converting θ and ψ values to measurements of blood and melanin concentration as is stored in the conversion table 17. In the case of pixels which are associated with null values of within the chromophore distribution data no θ and ψ values are determined.

By processing the chromophore distribution data in this way, and accessing the radial co-ordinates r for pixels generated by the spherical conversion module 10, the image generation module 18 is able to generate a derived image where each pixel image for which the conversion module 12 is able to determine chromophore distribution values is represented by a pair of calculated colour angles θ and ψ and a radial value r corresponding to the radial value for that particular pixel as determined by the spherical conversion module 15.

This derived image data is then passed to the geometry determination module 22 which proceeds to convert the array of received θ ψ r data into an image of equivalent RGB values.

This is achieved by applying the following equations to the θ ψ r data for each pixel:

$$R = r \sin \theta \cos \psi$$

$$G = r \sin \theta \sin \psi$$

$$B = r \cos \theta$$

The geometry determination module 22 then calculates (s3-5) a geometry term K for each pixel in the image using the following equation:

$$K = R_{original}/R_{derived}$$

Where $R_{original}$ is the red channel value for a pixel in the original image and $R_{derived}$ is the red channel value in the corresponding pixel in the derived image with K being set to a null value for all pixels for which no R,G,B data in the derived image could the calculated.

The differences between the derived image and the original image arise due to differences in the strength of illumination of the skin surface largely due to gross variations in the skin surface geometry.

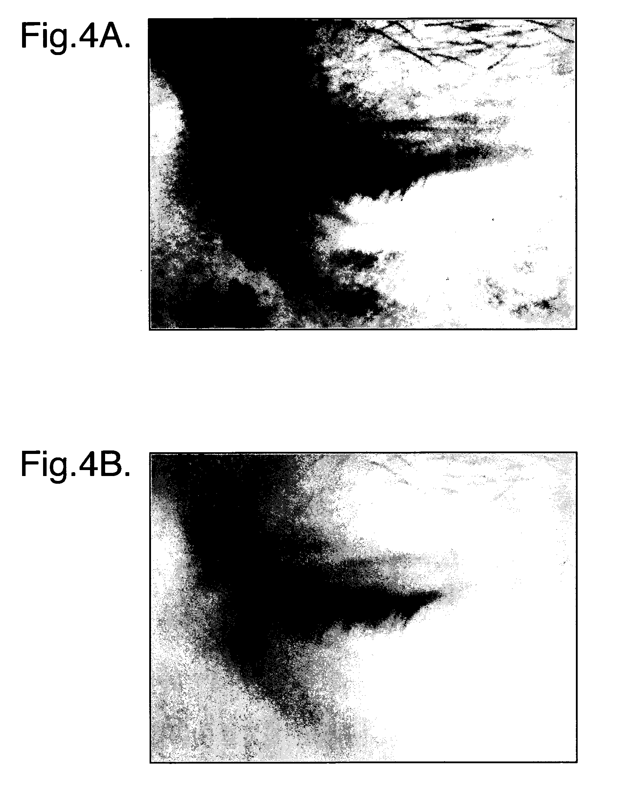

By way of example Figure 4A is an exemplary image of the eye of an individual. Figure 4B is an example of a corresponding image derived by determining an estimated blood and melanin distribution for the image of Figure 4A and then generating a derived image based on the expected appearance of the determined chromophore distribution together with spherical co-ordinate r values derived from the original image. As can be seen by in these two images around the eye portion as a result of the processing much of the gross surface geometry around the eye is lost.

Geometry data indicative of the extent that the variation in appearance arises due variation in illumination arising from the gross geometry of the surface being imaged independent of the presence of the concentrations of chromophores in the skin can therefore be obtained by calculating the ratios of pixel values for corresponding pixels in the original and derived images.

Returning to Figure 3, after geometry data indicative of lighting variations due to gross surface geometry has been determined (s3-5), a second image of the individual 2 is obtained (s3-6) by the digital camera 12. In contrast to the first image, this second image is obtained with the first polarizer 4 positioned so that the light source illuminates the individual 2 without passing through the polarizer 4. The obtained image will therefore indicate the appearance of the individual 2 based both on the reflection of light directly from the surface of the skin of the individual 2 and also due to the interaction of the light with the structures and chromophores present in the skin. This second image is then passed, together with the original image of the individual illuminated by polarized light and the calculated geometry data to the difference module 24.

When the images are received by the difference module 24, they are processed, together with the geometry data to generate (s3-7) a surface map for display on the display screen 10.

More specifically, initially, the difference in R value for each pixel in the second image compared with the corresponding R value for the corresponding pixel in the first image is determined. This enables a monochrome difference image to be calculated. Such a difference image is indicative the results of surface scatter by the skin. However, this data itself does not provide a measurement of skin texture because the difference image is also dependent upon variations in the strength of illumination. To account for this the determined difference values are therefore divided by the K value indicative of the variation in illumination due to gross surface geometry where this K value is available. Where only a null K value is available no surface measurement is determined. The rescaling of the difference values using the calculated K geometry terms effectively removes the variation arising due to variations in illumination and thus causes the resultant processed image to be representative of detailed variations in skin surface independent of the variation in gross surface geometry and illumination.

In the resultant surface map, pixels where little or non-surface reflection has occurred which will correspond to wrinkles or furrows in the skin will be associated with lower values with the relative size of the measurement indicative of the depth of the furrow or wrinkle. Additionally, the obtained map can also be used to measure the extent of areas of dry skin as such areas are associated with higher converted distance values and areas of surface maps indicative of areas having a greater variance of values.

Alternative Embodiments and Modifications

In the above embodiment, a system has been described where a polarizing filter 4 in front of a light source 3 is moveable between a first and second position to enable images of an individual 2 to be obtained illuminated by polarized or unpolarized light so that measurements of surface texture may be obtained. It will be appreciated that other alternative arrangements could be utilised to obtain these images.

Thus for example, instead of removing the polarizing filter 4 in front of the light source 3, the polarizing filter 5 in front of the camera 1 could be removed. Alternatively rather than moving either of the polarizing filters 4,5 either the camera 1 or the light source could be moved so as to obtain images without light passing through both of the polarizing filters 4,5.

In a preferred embodiment rather than removing one of the polarizing filters 4,5 one of the polarizing filters 4,5 could be rotated so that instead of being cross-polarized with the other, both filters 4,5 permitted light sharing the same polarization to pass. In such an embodiment, an image obtained with the filters 4,5 in such a configuration would comprise only light having the same polarization as the light with which the surface of the individual is illuminated. The difference between such an image and an image obtained with the filters 4,5 in a cross polarization configuration would therefore almost entirely arise due to variations caused by surface reflection and hence would be particularly suitable for determining a surface texture map by being normalized for variations in strength of illumination as determined in the manner described above.

As a further alternative to moving the positions or configurations of the polarizing filters 4,5 a pair of digital cameras could be provided to obtain an substantially identical images based on polarized light and unpolarized light respectively. Images obtained by the two cameras could then aligned and then processed in a similar way as has been described above. One advantage of such a system would be that polarized and unpolarized images could be obtained simultaneously.

In the above described embodiment the geometry term K is stated as being derived from differences in pixel values for the red channel. The difference in red channel values for the images obtained in the presence and absence of the polarizing filter are then rescaled utilising this term to normalize the difference image for differences in the strength of illumination arising due to large scale variations in surface geometry.

Utilising the red channel data in this way is preferable to utilising the other colour channels since red light is preferentially reflected by the skin and hence this data is more reliable than data for the other colour channels. In other embodiments, illumination level scaling factors could, however be obtained using any or a combination of any of the colour channels.

Although the embodiments of the invention described with reference to the drawings comprise computer apparatus and processes performed in computer apparatus, the invention also extends to computer programs, particularly computer programs on or in a carrier, adapted for putting the invention into practice. The program may be in the form of source or object code or in any other form suitable for use in the implementation of the processes according to the invention. The carrier can be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal which may be conveyed via electrical or optical cable or by radio or other means.

When a program is embodied in a signal which may be conveyed directly by a cable or other device or means, the carrier may be constituted by such cable or other device or means.

Alternatively, the carrier may be an integrated circuit in which the program is embedded, the integrated circuit being adapted for performing, or for use in the performance of, the relevant processes.

## Claims

1. A method of measuring skin surface texture comprising:

   illuminating an area of skin (2) with polarized light;
   obtaining (S3-1) a first measurement of light returned by the illuminated area of skin (2) wherein the measured light is light with a different polarity to the light with which said area of skin (2) is illuminated;
   processing (S3-2-S3-5) the obtained measurement of light utilising a model (15-22) of the interactions of light with chromophores present in the skin (2) to determine variations in returned light levels arising due to variations in levels of illumination;
   obtaining (s3-6) a second measurement of light returned by the illuminated area of skin (2) wherein the measured light includes light of the same polarity as the light with which said area of skin (2) is illuminated;
   utilising (s3-7) the difference between said first and said second measurements of light returned by the illuminated area of skin (2) and said determined variations in returned light levels arising due to variations in levels of illumination to obtain a measurement of the surface texture of the illuminated area of skin.

2. A method in accordance with claim 1, wherein processing (S3-2-S3-5) an obtained measurement of light utilising a model (15-22) of the interactions of light with chromophores present in the skin (2) to determine variations in returned light levels arising due to variations in levels of illumination comprises:

   processing (S3-2-S3-3) an obtained measurement of light to determine the concentrations of one or more chromophores in an illuminated area of skin (2);
   determining (S3-4) estimated expected levels of light arising due to the illumination of skin having said determined concentrations of chromophores; and
   determining (S3-5) variations in returned light levels arising due to variations in levels of illumination by comparing said estimated expected levels of light and an obtained measurement of light.

3. A method in accordance with claim 1 or claim 2 wherein said first (s3-1) and second (s3-6) measurements of light are obtained sequentially.

4. A method in accordance with claim 1 or claim 2 wherein said first (s3-1) and second (s3-6) measurements of light are obtained simultaneously.

5. A method in accordance with any preceding claim wherein said model (15-22) of the interactions of light with chromophores present in the skin (2) comprises a model of the interaction of light with one or more of haemoglobin and melanin.

6. An apparatus for measuring skin surface texture, the apparatus comprising:

a light source (3,4) operable to illuminate an area of skin (2) with polarized light;
a detector (1,5) operable to obtain a first and a second measurement of light returned by an illuminated area of skin (2) wherein the first measurement of light is light of a different polarity to the light with which said light source (3,4) is operable to illuminate said area of skin (2) and said second measurement of light includes light of the same polarity as the light by said light source (3,4) is operable to illuminate said area of skin (2); and
a processor (6, 15-24) operable to:

process (S3-2-S3-5) an obtained first measurement of light utilising a model (15-22) of the interactions of light with chromophores present in the skin (2) to determine variations in returned light levels arising due to variations in levels of illumination; and utilise (s3-7) the difference between detected first and said second measurements of light returned by the illuminated area of skin (2) and determined variations in returned light levels arising due to variations in levels of illumination to obtain a measurement of the surface texture of the illuminated area of skin (2).

7. An apparatus in accordance with claim 6 wherein said processor (6, 15-24) comprises:

a chromophore determination module (15-17) operable to process (S3-2-S3-3) an obtained measurement of light to determine the concentrations of one or more chromophores an illuminated area of skin (2);
an image generation module (18,20) operable to determine (S3-4) an estimated expected level of light arising due to the illumination of skin having said determined concentrations of one or more chromophores;

a geometry determination module (22) operable to determine (S3-5) variations in returned light levels arising due to variations in levels of illumination by comparing said estimated expected level of light and obtained measurement of light; and
a texture determination module (24) operable to utilise (s3-7) the difference between detected first and said second measurements of light returned by an illuminated area of skin (2) and determined variations in returned light levels arising due to variations in levels of illumination to obtain a measurement of the surface texture of the illuminated area of skin (2).

8. An apparatus in accordance with claim 6 or 7 wherein said light source (3,4) operable to illuminate an area of skin (2) with polarized light comprises a light source (3) operable to illuminate an area of skin (2) via a first polarizing filter (4).

9. An apparatus in accordance with claim 8, wherein said first polarizing filter (4) is movable between a first position in which said light source (3) is operable to illuminate an area of skin (2) via said first polarizing filter (4) and a second position in which said light source (3) is operable to illuminate an area of skin (2) in the absence said first polarizing filter (4).

10. An apparatus in accordance with any of claims 6 - 9 wherein said detector (1,5) comprises:

a digital camera (1) operable to obtain an image of an illuminated area of skin (2) via a second polarizing filter (5).

11. An apparatus in accordance with claim 10 wherein said second polarizing filter (5) is movable between a first position in which said digital camera (1) operable to obtain an image of an illuminated area of skin (2) via a second polarizing filter (5) and a second position in which said digital camera (1) operable to obtain an image of an illuminated area of skin (2) in the absence of said second polarizing filter (5) .

12. An apparatus in accordance with claim 10 wherein at least one of said first (4) or said second (5) polarizing filter is movable between a first position in which it is operable to filter light having the same polarity as filtered by the other polarizing filter and a second position in which it is operable to filter light not having the same polarity as the polarized light filtered by the other polarizing filter.

13. An apparatus in accordance with any of claims 6 - 8 wherein said detector (1,5) comprises a digital camera (1) operable to obtain an image of an illuminated area of skin (2) via a second polarizing filter (5) wherein said second polarizing filter (5) is movable between a first position in which it is operable to filter light having the same polarity as the polarized light generated by said light source (3,4) and a second position in which it is operable to filter light not having the same polarity as the polarized light generated by said light source (3,4).

14. An apparatus in accordance with claim 6 - 9 wherein said detector comprises:

a first detector operable to obtain a first measurement of light returned by an illuminated area of skin (2) wherein the first measurement of light is light of a different polarity to the light with which said light source (3,4) is operable to illuminate said area of skin (2);and
a second detector operable to obtain a second measurement of light returned by an illuminated area of skin (2) wherein the second measurement of light includes light of the same polarity as the light by said light source (3,4) is operable to illuminate said area of skin (2).

15. An apparatus in accordance with any of claims 6-14 wherein said model (15-22) of the interactions of light with chromophores present in the skin (2) comprises a model of the interactions of light with one or more of haemoglobin and melanin.

16. A computer readable medium (7,8) storing computer interpretable instructions which when processed by a programmable computer (6) cause the computer (6) to:

process (S3-2-S3-5) an obtained first measurement of light utilising a model (15-22) of the interactions of light with chromophores present in the skin (2) to determine variations in returned light levels arising due to variations in levels of illumination; and
utilise (s3-7) the difference between an obtained first and an obtained second measurement of light returned by an illuminated area of skin (2) and determined variations in returned light levels arising due to variations in levels of illumination to obtain a measurement of the surface texture of the illuminated area of skin (2).

17. A recording medium (7,8) in accordance with claim 16, wherein instructions which when processed by a programmable computer (6) cause the computer (6) to process(S3-2-S3-5) an obtained first measurement of light utilising a model (15-22) of the interactions of light with chromophores present in the skin (2) to determine variations in returned light levels arising due to variations in levels of illumination, comprise instructions which when processed by a programmable computer (6) cause the computer (6) to:

process (S3-2-S3-3) an obtained measurement of light to determine the concentrations of one or more chromophores an illuminated area of skin (2);
determine (S3-4) estimated expected levels of light arising due to the illumination of skin having said determined concentrations of one or more chromophores; and
determine (S3-5) variations in returned light levels arising due to variations in levels of illumination by comparing said estimated expected levels of light and an obtained measurement of light.

18. A recording medium (7,8) in accordance with claim 16 or 17, wherein said model (15-22) of the interactions of light with chromophores present in the skin (2) comprises a model of the interactions of light with one or more of haemoglobin and melanin.

19. A recording medium (7,8) in accordance with any of claims 16 - 18 comprising a computer disc (7).

20. A computer disc (7) in accordance with claim 19 comprising a magnetic, optical or magneto-optical disc.

21. A recording medium (7,8) in accordance with any of claims 16 - 18 comprising a signal (8) in a communications network.

Fig.1.

# Fig.2.

COMPUTER

Digital camera — 1

Light source — 3

RGB image data

Spherical conversion module — 15

Image conversion module — 16

Conversion table — 17

chromophore distribution

Image generation module — 18

Inverse conversion table — 20

$\theta \psi$   $r$

Derived image

Polarised image   Unpolarised image

Geometry determination module — 22

Geometry data

Difference module — 24

Surface Map

Display — 10

EP 2 005 885 A1

# Fig.3.

( START )

↓

| Obtain image data using polarised light | S3-1 |

↓

| Convert RGB image to spherical co-ordinates | S3-2 |

↓

| Determine chromophore distribution from $\theta$ & $\psi$ values | S3-3 |

↓

| Generate simulated image data using chromophore distribution | S3-4 |

↓

| Determine geometry data using original and simulated image data | S3-5 |

↓

| Obtain second image in the absence of polariser | S3-6 |

↓

| Determine surface map using polarised and unpolarised images and geometry data | S3-7 |

↓

( END )

Fig.4A.

Fig.4B.

European Patent
Office

**EUROPEAN SEARCH REPORT**

Application Number

EP 07 25 2478

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 768 060 A (ASTRON CLINICA LTD [GB]) 28 March 2007 (2007-03-28) * paragraphs [0014] - [0028], [0033] - [0039] * | 1,6,16 | INV. A61B5/103 |
| A | RAMELLA-ROMAN J C ET AL: "Out-of-plane polarimetric imaging of skin: surface and subsurface effects" PROCEEDINGS OF THE SPIE, SPIE, BELLINGHAM, VA, US, vol. 5686, no. 1, April 2005 (2005-04), pages 142-153, XP002386128 ISSN: 0277-786X * the whole document * | 1,6 | |

TECHNICAL FIELDS
SEARCHED      (IPC)

A61B

The present search report has been drawn up for all claims

| Place of search | Date of completion of the search | Examiner |
|---|---|---|
| Munich | 14 November 2007 | Manschot, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

# EP 2 005 885 A1

**ANNEX TO THE EUROPEAN SEARCH REPORT
ON EUROPEAN PATENT APPLICATION NO.**

EP 07 25 2478

This annex lists the patent family members relating to the patent documents cited in  the above-mentioned European search report.
The members are as contained in the European Patent Office EDP file on
The European Patent Office is in no way liable for these particulars which are merely given for the purpose of information.

14-11-2007

| Patent document cited in search report | | Publication date | Patent family member(s) | | Publication date |
|---|---|---|---|---|---|
| EP 1768060 | A | 28-03-2007 | AU | 2006220441 A1 | 19-04-2007 |
| | | | CA | 2560364 A1 | 23-03-2007 |
| | | | GB | 2429385 A | 21-02-2007 |
| | | | JP | 2007190364 A | 02-08-2007 |

EPO FORM P0459

For more details about this annex : see Official Journal of the European Patent Office, No. 12/82

**14**